# EUROPEAN PATENT APPLICATION

(11) **EP 2 756 843 A2**
(43) Date of publication of application: **23.07.2014**
(21) Application number: 14158789.9
(22) Date of filing: 25.07.2006
(51) Int. Cl.: A61K 31/216, A61P 29/00, A61P 43/00, C07C 229/42, C07C 237/20

(54) **Positively charged water-soluble prodrugs of diclofenac with very fast skin penetration rate**

(62) Divisional of application: 06780202.5
(71) Applicant: Techfields Biochem Co. Ltd, Shanghai N/A 200444 (CN); Yu, Chongxi, Plainfield, Illinois 60585 (US)
(72) Inventor: Yu, Chongxi, Plainfield, IL Illinois 60585 (US); Xu, Lina, 200444 Shanghai (CN)
(74) Representative: Viering, Jentschura & Partner Patent- und Rechtsanwälte

(57) **Abstract**

The novel positively charged pro-drugs of diclofenac in the general formula (1), with X = O or S, were designed and synthesized. The compounds of the general formula (1) can be prepared from functional derivatives of diclofenac by reaction with suitable alcohols, thiols, or amines. The positively charged amino groups of these pro-drugs not only largely increase the solubility of the drugs in water, but also bond to the negative charge on the phosphate head group of membranes and push the pro-drug into the cytosol. Experimental results suggest that the pro-drug diethylaminoethyl 2[(2,6-dichlorophenyl)amino]benzene acetate.AcOH diffuses through human skin 250 times faster than 2[(2,6-dichlorophenyl)amino]benzene acetic acid (diclofenac) and ethyl 2[(2,6-dichlorophenyl)amino]benzene acetate. In plasma, more than 90% of these pro-drugs can change back to the drug in a few minutes. The prodrugs can be used medicinally in treating any diclofenac-treatable conditions in humans or animals and be administered not only orally, but also transdermally for any kind of medical treatments and avoid most of the side effects of diclofenac, most notably GI disturbances such as dyspepsia, gastroduodenal bleeding, gastric ulcerations, and gastritis. Controlled transdermal administration systems of the prodrug enables diclofenac to reach constantly optimal therapeutic blood levels to increase effectiveness and reduce the side effects of diclofenac.

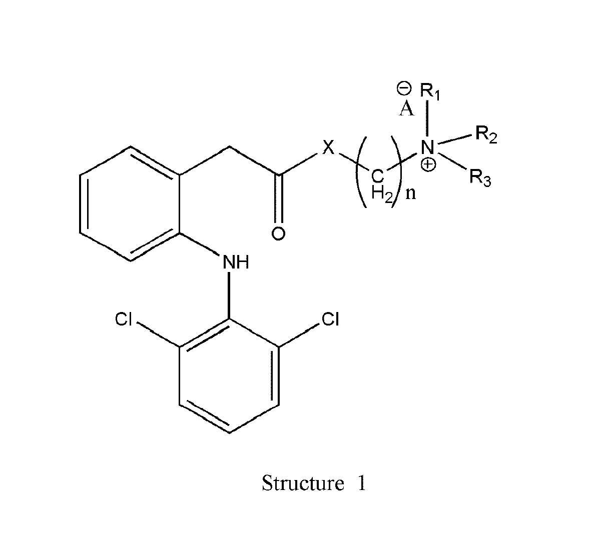

## Description

### Technical Field

The present invention relates to the preparations of positively charged and water-soluble pro-drugs of 2[(2, 6-dichlorophenyl) amino] benzene acetic acid (diclofenac) and their medicinal use in treating any diclofenac-treatable conditions in humans or animals. More specifically, the present invention is to overcome the side effects that are associated with the use of diclofenac. These pro-drugs can be administered orally or transdermally.

### Background Art

Diclofenac is a member of the aryl- and heteroarylacetic acid group of nonsteroidal anti-inflammatory drugs. The synthesis of diclofenac was originally reported in 1969 (A. Sallman and R. Pfister, Ger. Patent No. 1,815,802). Diclofenac is available in 120 different countries and is perhaps the most widely used NSAIA in the world. Diclofenac possesses structural characteristics of both the arylalkanoic acid and the anthranilic acid classes of anti-inflammatory agents and displays anti-inflammatory, analgesic, and antipyretic properties. As an analgesic, it is 6 times more potent than indomethacin and 40 times more potent than aspirin. As an antipyretic, it is twice as potent as indomethacin and over 350 times as potent as aspirin. 'PDR Generics' (PDR Generics, 1996, second edition, Medical Economics, Montvale, New Jersey, pg 243) has listed many medical uses of diclofenac. Diclofenac is used for the relief of signs and symptoms of rheumatoid arthritis, osteoarthritis and ankylosing spondylitis. Its potassium salt is indicated for the treatment of dysmenorrhea. Diclofenac is used alone or as an adjunct in the treatment of biliary colic, fever, and episiotomy pain.. It is also used in treatment of gout, acute migraine headaches, and renal colic and in the treatment of postoperative inflammation in patients who have undergone cataract extraction.

Unfortunately, a number of side effects are associated with the use of diclofenac, most notably GI disturbances such as dyspepsia, gastroduodenal bleeding, gastric ulcerations, and gastritis. Fishman (Fishman; Robert, U.S. Pat. No. 7,052,715) indicated that an additional problem associated with oral medications, is that the concentration levels which must be achieved in the bloodstream must be significant in order to effectively treat distal areas of pain or inflammation. These levels are often much higher than would be necessary if it were possible to accurately target the particular site of pain or injury. Fishman and many others (Van Engelen et al. U.S. Pat. No. 6,416,772; Macrides et al. U.S. Pat. No. 6,346,278; Kirby et al. U.S. Pat. No. 6,444,234, Pearson et al. U.S. Pat. No. 6,528,040, and Botknecht et al. U.S. Pat. No. 5,885,597) have attempted to develop a delivery system for transdermal application by formulation. Song, et al. developed a transdermal drug delivery system for anti-inflammatory analgesic agent comprising diclofenac diethylammonium salt (Song, et. al., US Patent No. 6,723,337). Donati, et al. developed a plaster for topical use containing heparin and diclofenac. (Donati, et al., US patent, NO. 6,592,891). Kawaji, et al. developed an oily patch for external use containing diclofenac sodium (Kawaji, et al. US Patent No. 6,262,121). Effing, et al. developed a device for the transdermal delivery of diclofenac (Effing, et al. US Patent No. 6,193,996). It is very difficult, however, to deliver therapeutically effective plasma levels of diclofenac into the host by formulation. Susan Milosovich, et. al. designed and prepared testosteronyl-4- dimethylaminobutyrate.HCl (TSBH), which has a lipophilic portion and a tertiary amine groups that exists in the protonated form at physiological pH. They found that the prodrug (TSBH) diffuses through human skin -60 times faster than does the drug (TS) itself [Susan Milosovich, et al., J. Pharm. Sci., 82, 227(1993).

### Disclosure of Invention

### Technical Problem

Diclofenac has been used medicinally for more than 30 years and diclofenac is more potent than aspirin in anti-inflammatory and prostaglandin biosynthesis inhibition. Diclofenac is indicated for the relief of the signs and symptoms of rheumatoid arthritis and osteoarthritis, the relief of mild to moderate pain, the reduction of fever, and the treatment of dysmenorrhea. Diclofenac is available in 120 different countries and is perhaps the most widely used NSAIA in the world.

Unfortunately, a number of side effects are associated with the use of diclofenac, most notably GI disturbances such as dyspepsia, heartburn, vomiting, gastroduodenal bleeding, gastric ulcerations, and gastritis. Gastroduodenal bleeding induced by diclofenac is generally painless but can lead to fecal blood loss and may cause a persistent iron deficiency anemia.

### Technical Solution

This invention relates to the preparation of novel positively charged pro-drugs of diclofenac and their use medicinally. These pro-drugs have the general formula (1) 'Structure 1'.

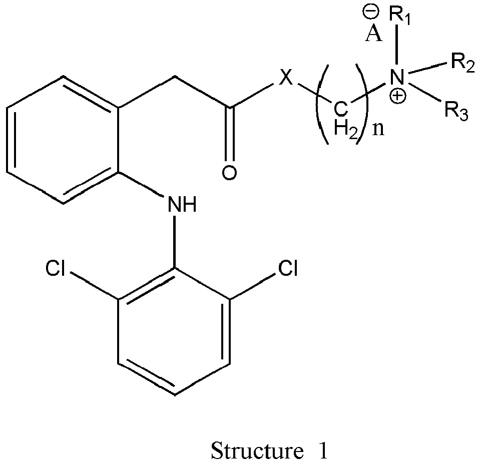

In structure 1, R represents H, one of any alkyl, alkyl, alkenyl or alkynyl residues having 1 to 12 carbon atoms, or aryl residues; R₂ represents H, one of any alkyl, alkyloxy, alkenyl or alkynyl residues having 1 to 12 carbon atoms, or aryl residues; R₃ represents H, one of any alkyl, alkyloxy, alkenyl or alkynyl residues having 1 to 12 carbon atoms, or aryl residues; X represents O, S or NH; A⁻ represents Cl⁻, Br⁻, F⁻, I⁻, AcO⁻, citrate, or any negative ions; and n=0,1,2,3,4,5,6,7,8,9,10...... All R groups may include C, H, O, S, N atoms and may have single, double, and treble bonds. Any CH₂ groups may be replaced with O, S, or NH.

Drug absorption, whether from the gastrointestinal tract or other sites, requires the passage of the drug in a molecular form across the barrier membrane. The drug must first dissolve, and if the drug possesses the desirable biopharmaceutical properties, it will pass from a region of high concentration to a region of low concentration across the membrane into the blood or general circulation. All biological membranes contain lipids as major constituents. The molecules that play the dominant roles in membrane formation all have phosphate-containing highly polar head groups, and, in most cases, two highly hydrophobic hydrocarbon tails. Membranes are bilayers, with the hydrophilic head groups facing outward into the aqueous regions on either side. Very hydrophilic drugs cannot pass the hydrophobic layer of membrane and very hydrophobic drugs will stay in the hydrophobic layer as part of the membrane due to their similarities and cannot enter the cytosol on the inside efficiently.

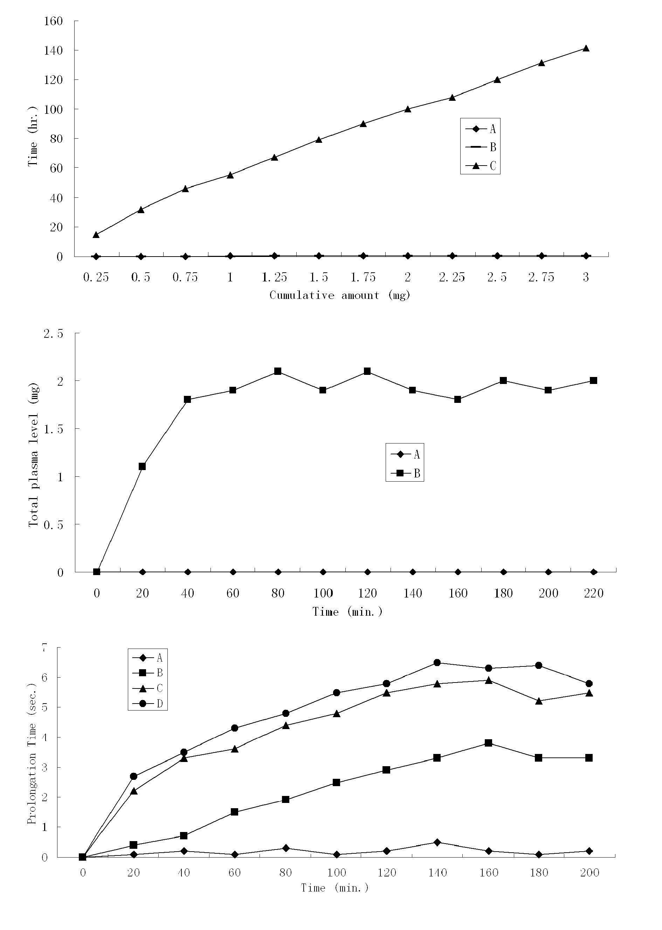

The goal of this invention is to avoid the side effects of diclofenac by increasing the solubility of diclofenac in gastric juice and the penetration rate of diclofenac through the membrane and skin barrier which will make it administrable transdermally (topical application). These novel pro-drugs of diclofenac have two structural features in common: they have a lipophilic portion and a primary, secondary, or tertiary amine group that exists in the protonated form (hydrophilic part) at physiological pH. Such a hydrophilic-lipophilic balance is required for efficient passage through the membrane barrier [Susan Milosovich, et al., J. Pharm. Sci., 82, 227(1993)]. The positively charged amino groups largely increase the solubility of the drugs. The solubility of diethylaminoethyl 2[(2,6-dichlorophenyl)amino]benzene acetate.AcOH and 2[(2,6-dichlorophenyl)amino]benzene acetic acid (diclofenac) in water were >400 mg and 0.1 mg/ml. In many instances, the slowest or rate-limiting step in the sequence is the dissolution of the drug. Diclofenac has a very low solubility in gastric juice. It stays in the GI tract for a long time and thus, may cause gastric mucosal cell damage. When these new pro-drugs are administered orally in a dosage form such as a tablet, capsule, solution, or suspension, they will dissolve in the gastric juice immediately. The positive charge on the amino groups of these pro-drugs will bond to the negative charge on the phosphate head group of membrane. Thus, the local concentration of the outside of the membrane will be very high and will facilitate the passage of these pro-drugs from a region of high concentration to a region of low concentration. When these pro-drugs enter the membrane, the hydrophilic part of the pro-drugs will push the pro-drug into the cytosol, a semi-liquid concentrated aqueous solution or suspension. Due to the short stay in GI tract, the pro-drugs will not cause gastric mucosal cell damage. The penetration rates of diethylaminoethyl 2[(2,6-dichlorophenyl)amino]benzene acetate.AcOH and 2[(2,6-dichlorophenyl)amino]benzene acetic acid (diclofenac) through human skin were measured in vitro by using modified Franz cells, which were isolated from human skin tissue (360-400 µm thick) of the anterior and posterior thigh areas. The receiving fluid consisted of 10 ml of 2% bovine serum albumin in normal saline and was stirred at 600 rpm. The cumulative amounts of diethylaminoethyl 2[(2,6-dichlorophenyl)amino]benzene acetate.AcOH and 2[(2,6-dichlorophenyl)amino ]benzene acetic acid (diclofenac) penetrating the skin versus time were determined by a specific high-performance liquid chromatography method. The results using a donor consisting of either a 30% suspension of 2[(2,6-dichlorophenyl)amino]benzene acetic acid (diclofenac) and ethyl 2[(2,6-dichlorophenyl)amino]benzene acetate and 30% solution of diethylaminoethyl 2[(2,6-dichlorophenyl)amino]benzene acetate.AcOH in 2mL of pH 7.4 phosphate buffer (0.2M) are shown in Figure 1. Apparent flux values of 0.2 mg, 0.2 mg and 50 mg/cm²/h were calculated for 2[(2,6-dichlorophenyl)amino] benzene acetic acid (diclofenac), ethyl 2[(2,6-dichlorophenyl)amino]benzene acetate (the non positive charged normal ester of diclofenac) and diethylaminoethyl 2[(2,6-dichlorophenyl)amino]benzene acetate.AcOH. The results suggest that the pro-drug, diethylaminoethyl 2[(2,6-dichlorophenyl)amino]benzene acetate.AcOH diffuses through human skin -250 times faster than do 2[(2,6-dichlorophenyl)amino]benzene acetic acid (diclofenac) and ethyl 2[(2,6-dichlorophenyl)amino]benzene acetate. The normal ester, ethyl 2[(2,6-dichlorophenyl)amino]benzene acetate and diclofenac itself have same penetration rate. The results suggest that the positive charge on the dialkyaminoethyl group has a very important role in the passage of the drug across the membrane and skin barrier. Other prodrugs of the general 'Structure 1' have very high penetration rates and are very close to that of diethylaminoethyl 2[(2,6-dichlorophenyl)amino]benzene acetate.AcOH.

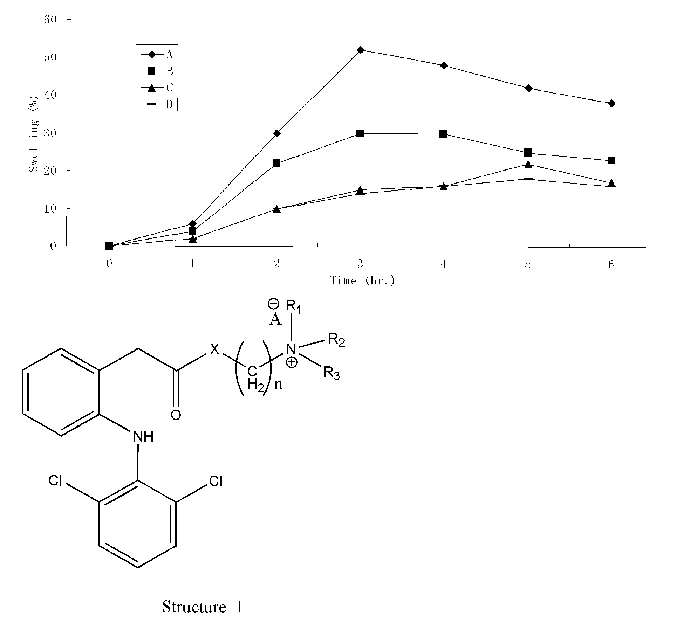

The in vivo rates of penetration of diethylaminoethyl 2[(2,6-dichlorophenyl)amino] benzene acetate.AcOH and 2[(2,6-dichlorophenyl)amino]benzene acetic acid (diclofenac) through the skin of intact hairless mice were compared. The donor consisted of a 20% solution of diethylaminoethyl 2[(2,6-dichlorophenyl)amino] benzene acetate.AcOH and 2[(2,6-dichlorophenyl)amino]benzene acetic acid (diclofenac) in 1 mL of isopropanol applied to a 1 cm² on the backs of the hairless mice. The results (Figure 2) show that the peak levels of diclofenac was reached -40 minutes after application of the donor systems. It takes 1-2 hours for diclofenac to reach the peak diclofenac level when it is taken orally. The peaks were -0.001 mg/ml for diclofenac or -2.1 mg/ml for diethylaminoethyl 2[(2,6-dichlorophenyl)amino] benzene acetate.AcOH (approximately 2100 times difference). -2.1 mg/ml of diclofenac in plasma is more than 1000 times higher than the diclofenac plasma level for effective analgesia and effective anti-inflammatory activity (-0.002 mg/ml). This is a very exciting result. It will be very easy and fast to deliver therapeutically effective plasma levels of diclofenac into the host by these pro-drugs. These results suggest that the pro-drugs can be administered not only orally, but also transdermally for any kind of medical treatments. The in vivo rates of penetration of other pro-drugs of the general 'Structure 1' are close to that of diethylaminoethyl 2[(2,6-dichlorophenyl)amino]benzene acetate.AcOH.

To check the gastroduodenal bleeding caused by drugs, rats (two groups, each group had 10 rats) were orally administered with 25 mg/kg of diclofenac or diethylaminoethyl 2[(2,6-dichlorophenyl)amino]benzene acetate.AcOH per day for 21 days. We found an average of 3 mg of fecal blood per gram of feces in the diclofenac group and none in diethylaminoethyl 2[(2,6-dichlorophenyl)amino]benzene acetate.AcOH group.

The acute toxicity of the prodrugs was investigated. The LD₅₀ orally in rats are: 0.75 g/kg and 0.7 g for diethylaminoethyl 2[(2,6-dichlorophenyl)amino]benzene acetate.AcOH and dimethylaminoethyl 2[(2,6-dichlorophenyl)amino]benzene acetate.AcOH . The results show that the prodrugs are less toxic than diclofenac (LD₅₀ =0.45g/kg).

Diclofenac has demonstrated anti-inflammatory, analgesic, antipyretic, and antirheumatic activity. A good prodrug should go back to the drug itself in plasma. Diethylaminoethyl ester group of diethylaminoethyl 2[(2,6-dichlorophenyl)amino] benzene acetate.AcOH can be rapidly cleaved by the enzymes in human plasma in vitro and more than 90% of the pro-drug is changed back to diclofenac. Due to the prodrug having a much better absorption rate, the prodrug will have more strength than the diclofenac itself at the same dosage. The analgetic, antipyretic, and anti-inflammatory activities of diethylaminoethyl 2[(2,6-dichlorophenyl)amino]benzene acetate.AcOH were tested using diclofenac as a comparison. Other compounds of the general 'Structure 1' were tested by the same methods and have very similar results as that of diethylaminoethyl 2[(2,6-dichlorophenyl)amino]benzene acetate.AcOH.

Analgetic activity: The prolongation time of pain the threshold of a mouse tail was determined in accordance with the D'Amour-Smith Method (J. Pharmacol. Exp. Ther., 72, 74(1941)). After25 mg/kg of diclofenac was administered orally and 25 mg/kg of diethylaminoethyl 2[(2,6-dichlorophenyl)amino]benzene acetate.AcOH was administered orally and transdermally, the tails of mice were exposed to heat and the prolongation time of pain threshold was determined. The results obtained are shown in Figure 3. The groups administered 25 mg/kg of diethylaminoethyl 2[(2,6-dichlorophenyl)amino]benzene acetate.AcOH orally (C) and transdermally (D) were shown to exhibit stronger analgetic activity than the group administered 25 mg/kg of diclofenac.

The number of writhings that occurred when mice were administered an acetic acid solution intraperitoneally were counted, and the rate of inhibition based on the control group was calculated. 42 mice were divided into 7 groups (6 mice each). Diclofenac (10 mg and 20 mg/kg) was administered to groups B1 and B2 of mice and diethylaminoethyl 2[(2,6-dichlorophenyl)amino]benzene acetate.AcOH (10 mg and 20 mg/ kg) was administered orally to groups C1 and C2. Diethylaminoethyl 2[(2,6-dichlorophenyl)amino]benzene acetate.AcOH (10 mg and 20 mg/kg) was administered transdermally to groups D1 and D2. The A group is the control group. The test compounds were administered to the mice 30 minutes before the acetic acid solution was administered. The results are shown in Table 1.

**Table 1. The rate of writhings inhibition by diclofenac and its pro-drugs**

| Group | A | B1 | B2 | C1 | C2 | D1 | D2 |
|---|---|---|---|---|---|---|---|
| Dose (mg/kg) | 0 | 10 | 20 | 10 | 20 | 10 | 20 |
| No. of writhings | 34.2 | 14.2 | 10.1 | 12.1 | 9.2 | 10.3 | 8.8 |
| % | - | 58.5 | 70.5 | 64.6 | 73.1 | 69.9 | 74.3 |

The results show that diethylaminoethyl 2[(2,6-dichlorophenyl)amino]benzene acetate.AcOH demonstrates better analgetic activity than diclofenac. Other compounds of the general 'Structure 1' show similar analgetic activity.

Antipyretic activity: Rats received a sterilized E. coli suspension as a pyrogen. 56 rats were divided into 7 groups. The control group is group A. 2 hours later, diclofenac (B1 for 10 mg/kg and B2 for 20 mg/kg) was administered orally and diethylaminoethyl 2[(2,6-dichlorophenyl)amino]benzene acetate.AcOH was administered orally (C1 for 10 mg/kg and C2 for 20 mg/kg) and transdermally (D1 for 10 mg/kg and D2 for 20 mg/kg). The body temperatures of rats were taken at 90 min. intervals before and after the administration of the test compounds. The results are shown in Table 2.

**Table 2. Antipyretic activity of diclofenac and its pro-drugs**

| Compound | t=0 min. | t=90 min. | t=180 min. | t=270 min. |
|---|---|---|---|---|
| Control group(A) | 37.56±0.05 | 37.55±0.07 | 37.53±0.05 | 37.52±0.08 |
| (10mg/kg, B1) | 37.56±0.06 | 36.90±0.05 | 36.91±0.08 | 36.92±0.07 |
| (20mg/kg, B2) | 37.55±0.09 | 36.60±0.07 | 36.53±0.06 | 36.55±0.05 |
| (10mg/kg, C1, orally) | 37.52±0.07 | 36.50±0.06 | 36.60±0.05 | 36.55±0.08 |
| (20mg/kg, C2, orally) | 37.54±0.08 | 36.30±0.05 | 36.35±0.07 | 36.38±0.08 |
| (10mg/kg, D1, transdermally) | 37.58±0.06 | 36.30±0.06 | 36.35±0.08 | 36.31±0.07 |
| (20mg/kg, D2, transdermally) | 37.59±0.05 | 36.25±0.05 | 36.30±0.07 | 36.20±0.05 |

The results shown that diethylaminoethyl 2[(2,6-dichlorophenyl)amino]benzene acetate.AcOH demonstrated antipyretic activity at 10 mg/kg and 20 mg/kg dose and better than that of diclofenac. The results show that transdermal administration of diethylaminoethyl 2[(2,6-dichlorophenyl)amino]benzene acetate.AcOH is better than oral administration. Other compounds of the general 'Structure 1' show similar antipyretic activity.

Anti-inflammatory activity: 10 mg/kg of diethylaminoethyl 2[(2,6-dichlorophenyl)amino]benzene acetate.AcOH was administered orally or transdermally to rats and 10 mg/kg of diclofenac was administered orally. 60 minutes later, a carrageenin solution was administered subcutaneously to the foot pads of the rats. The volume of the hind paw was measured at every hour after the administration of the carrageenin, and the rate of increase in the volume of the paw was calculated and designated as the rate of swelling (%). The results obtained are shown in Figure 4. The results show that diethylaminoethyl 2[(2,6-dichlorophenyl)amino] benzene acetate.AcOH by oral administration and transdermal administration demonstrated better Anti-inflammatory activity than that of diclofenac at 10 mg/kg by oral administration. Other compounds of the general 'Structure 1' show similar anti-inflammatory activity.

The compounds of the general formula (1) 'Structure 1' indicated above can be prepared from diclofenac or from functional derivatives of diclofenac. For example, acid halides or mixed anhydrides of the general formula (2) 'Structure 2'. 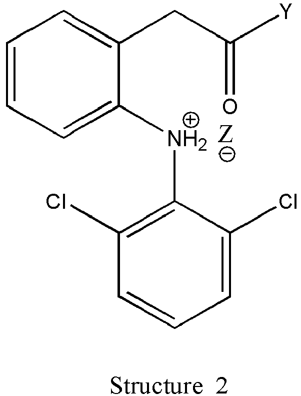 In structure 2, Y represents halogen, alkoxycarbonyl or substituted aryloxy-carbonyloxy, Z represents halogen or other negative ions, by reaction with compounds of the general formula (3) 'Structure 3', 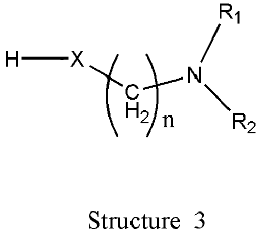 In structure 3, R₁ represents H, one of any alkyl, alkyloxy, alkenyl, or alkynyl residues having 1 to 12 carbon atoms, or aryl residues; R₂ represents H, one of any alkyl, alkyloxy, alkenyl or alkynyl residues having 1 to 12 carbon atoms, or aryl residues; X represents O, S or NH; and n=0,1,2,3,4,5,6,7,8,9,10 .......

The compounds of the general formula (1) 'Structure 1' indicated above can be prepared from diclofenac, by reaction with compounds of the general formula (3) 'Structure 3' by using coupling reagents, such as N,N'-Dicyclohexylcarbodiimide, N, N'-Diisopropylcarbodiimide, O-(Benzotriazol-1-yl)-N,N,N',N'-tetramethyluronium tetrafluoroborate, O-(Benzotriazol-1-yl)-N,N,N',N'-tetramethyluronium hexafluorophosphate, Benzotriazol-1-yl-oxy-tris (dimethylamino)phosphonium hexafluorophosphate, et al.

When X represents O, the compounds of the general formula (1) 'Structure 1' indicated above can be prepared from metal salts or organic base salts of diclofenac, by reaction with compounds of the general formula (4) 'Structure 4'. 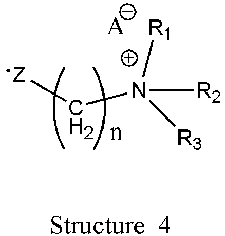 In structure 4, R₁ represents H, one of any alkyl, alkyloxy, alkenyl, or alkynyl residues having 1 to 12 carbon atoms, or aryl residues; R₂ represents H, one of any alkyl, alkyloxy, alkenyl, or alkynyl residues having 1 to 12 carbon atoms, or aryl residues; R₃ represents H, one of any alkyl, alkyloxy, alkenyl, or alkynyl residues having 1 to 12 carbon atoms, or aryl residues; Z represents halogen, or p-toluenesulphonyl, A⁻ represents Cl⁻, Br⁻, F, I⁻, AcO⁻, citrate, or any negative ions; and n=0,1,2,3,4,5,6,7,8,9,10.......

When X represents O, the compounds of the general formula (1) 'Structure 1' indicated above can be prepared from immobilized base salts of diclofenac of the general formula (5) 'Structure 5', 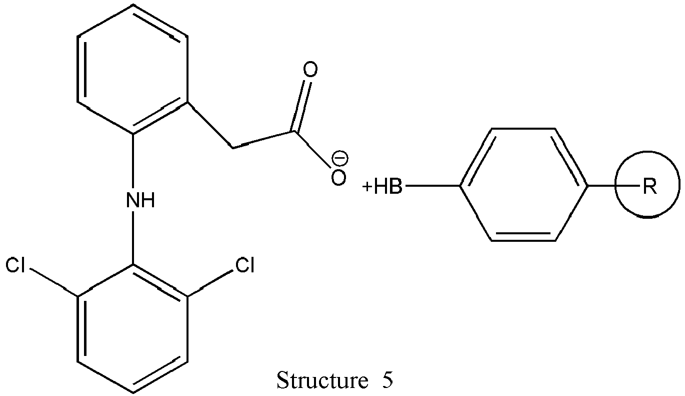 In structure 5, R represents cross-linked resin; B represents any base groups, such as pyridine, piperidine, triethylamine, or other base groups, by reaction with compounds of the general formula (4) 'Structure 4'.

The present invention relates to pharmaceutical preparations comprising of prodrugs of diclofenac of the general 'Structure 1' in addition to customary auxiliaries and excipients, in the form of tablets, capsules or solutions for administration orally and in the form of solutions, lotion, ointment, emulsion or gel for administration transdermally. The new active compounds of the general 'Structure 1' can be combined with vitamins such as A, B, C or E or beta-carotene, or other pharmaceuticals, such as folic acid, etc. for treating any diclofenac-treatable conditions in humans or animals.

It is also known that diclofenac shows an anti-reactive-antiasthmatic activity by inhibition of the cyclooxygenase activity. Due to their very high membrane penetration rate, these pro-drugs can be used in treating asthma by spraying into the mouth or nose of the host.

They can also be used to treat acne and other skin disorders due to their anti-inflammatory properties. They can be used for the treatment and prevention of endothelia dysfunction as well.

These pro-drugs are water-soluble neutral salt and can be tolerated very well by the eye. They can be used for treating eye inflammatory diseases, for treating of ocular pain after corneal surgery, for treating glaucoma or for treating ear inflammatory and/ or painful conditions (otitis).

Transdermal therapeutic application systems of compounds of the general 'Structure 1' or a composition comprising of at least one compound of the general 'Structure 1,' as an active ingredient, can be used for treating any diclofenac-treatable conditions in humans or animals. These systems can be a bandage or a patch comprising of one active substance-containing matrix layer and an impermeable backing layer. The most preferable system is an active substance reservoir, which has a permeable bottom facing the skin. By controlling the rate of release, this system enables diclofenac to reach constantly optimal therapeutic blood levels to increase effectiveness and reduce the side effects of diclofenac. These systems can be worn on the wrist, ankle, arm, leg, or any part of body.

### Advantageous Effects

These pro-drugs of diclofenac have a lipophilic portion and a hydrophilic portion (the amine groups that exist in the protonated form at physiological pH). The positively charged amino groups of these pro-drugs have two major advantages. First, it largely increases the solubility of the drugs in water; when these new pro-drugs are administered orally in a dosage form such as a tablet, capsule, solution, or suspension, they will dissolve in gastric juice immediately. Second, the positive charge on the amino group of these pro-drugs will bond to the negative charge on the phosphate head group of membrane. Thus, the local concentration outside of the membrane will be very high and will facilitate the passage of these pro-drugs from a region of high concentration to a region of low concentration. When these pro-drugs enter the membrane, the hydrophilic part will push the pro-drugs into the cytosol, a semi-liquid concentrated aqueous solution or suspension. Due to the short stay in the GI tract, the pro-drugs will not cause gastric mucosal cell damage. Experiment results show that more than 90% of the pro-drug was changed back to the drug itself. The pro-drugs have a much better absorption rate, and thus the pro-drugs will have better strength than the diclofenac itself at the same dosage. The experiment results suggest that the pro-drug, diethylaminoethyl 2[(2,6-dichlorophenyl)amino]benzene acetate.AcOH, diffuses through human skin -250 times faster than diclofenac itself and ethyl 2[(2,6-dichlorophenyl)amino]benzene acetate. The in vivo rates of penetration of diethylaminoethyl 2[(2,6-dichlorophenyl)amino]benzene acetate.AcOH through the skin of intact hairless mice were very high. It takes 1-2 hours for diclofenac tablets to reach the peak diclofenac plasma level when it is taken orally, but diethylaminoethyl 2[(2,6-dichlorophenyl)amino]benzene acetate.AcOH only took about 30 minutes to reach the peak diclofenac plasma level. The most exciting result is that the pro-drugs can be administered not only orally, but also transdermally for any type of medical treatment and should avoid most of the side effects of diclofenac, most notably GI disturbances such as dyspepsia, gastroduodenal bleeding, gastric ulcerations, and gastritis. Another great benefit of transdermal administration of these pro-drugs is that administering medication, especially to children, will be much easier.

### Description of Drawings

Figure 1: Cumulative amounts of 2[(2,6-dichlorophenyl)amino]benzene acetic acid (diclofenac, A), ethyl 2[(2,6-dichlorophenyl)amino]benzene acetate (the non positive charged normal ester of diclofenac, B) and diethylaminoethyl 2[(2,6-dichlorophenyl)amino]benzene acetate.AcOH (C) crossing isolated human skin tissue in Franz cells (n=5). Diclofenac and ethyl 2[(2,6-dichlorophenyl)amino]benzene acetate were applied as a 30% suspension; diethylaminoethyl 2[(2,6-dichlorophenyl)amino]benzene acetate.AcOH was applied as 30% solution. In each case, the vehicle was pH 7.4 phosphate buffer (0.2 M).
Figure 2: Total plasma levels of diclofenac after topical application of 1 ml of a 20% solution of diethylaminoethyl 2[(2,6-dichlorophenyl)amino]benzene acetate.AcOH and 2[(2,6-dichlorophenyl)amino]benzene acetic acid (diclofenac) in isopropanol to the backs of hairless mice (n=5).
Figure 3: The prolongation time of the pain threshold of mice tails after 25 mg/kg of diclofenac (B) was administered orally, 25 mg/kg of diethylaminoethyl 2[(2,6-dichlorophenyl)amino]benzene acetate.AcOH was administered orally (C) and transdermally (D). A is the control line.
Figure 4. The rate of swelling (%) after a carrageenin injection. 1 hour before the carrageenin injection, 10 mg/kg of diclofenac was administered orally (B), 10 mg/kg of diethylaminoethyl 2[(2,6-dichlorophenyl)amino]benzene acetate.AcOH was administered orally (C) and transdermally (D). A group is the control group.
Structure 1: In structure 1, R₁ represents H, one of any alkyl, alkyl, alkenyl or alkynyl residues having 1 to 12 carbon atoms, or aryl residues; R₂ represents H, one of any alkyl, alkyloxy, alkenyl or alkynyl residues having 1 to 12 carbon atoms, or aryl residues; R₃ represents H, one of any alkyl, alkyloxy, alkenyl or alkynyl residues having 1 to 12 carbon atoms, or aryl residues; X represents O, S or NH; A⁻ represents Cl⁻, Br⁻, F⁻, I⁻, AcO⁻, citrate, or any negative ions; and n=0,1,2,3,4,5,6,7,8,9,10...... All R groups may include C, H, O, S, N atoms and may have single, double, and treble bonds. Any CH₂ groups may be replaced with O, S, or NH.

### Best Mode

### Preparation of diethylaminoethyl 2[(2,6-dichlorophenyl)amino]benzene acetate.AcOH

35.1 g (0.1 mol) of 2[(2,6-dichlorophenyl)amino]benzene acetyl chloride hydrochloride was dissolved in 100 ml of chloroform. The mixture was cooled to 0°C. 30 ml of triethylamine and 11.7 g of diethylaminoethanol were added into the reaction mixture. The mixture is stirred for 3 hours at RT. The solid side product was removed by filtration and washed with chloroform (3 x 30 ml). 6 g of acetic acid is added into the reaction mixture with stirring. The organic solution was evaporated off. After drying, it yielded 39 g of the desired product (85.6 %). Hygroscopic product; Solubility in water: 400 mg/ml; Elementary analysis: C₂₂H₂₈Cl₂N₂O₄; MW: 455.37. Calculated % C: 58.03; H: 6.20; Cl: 15.57; N: 6.15; O: 14.05; Found % C: 58.01; H: 6.22; Cl: 15.55, N: 6.14; O: 14.09. ¹H-NMR (400 MHz, CDCl₃): δ: 1.56 (t, 6H), 2.21 (s, 3H), 3.28 (m, 4H), 3.50 (s, 2 H), 3.52 (m, 2H), 3.81 (b, 1H), 4.51 (t, 2H), 6.32 (d, 1H), 6.50 (m, 2 H), 6.78 (b, 1H), 6.82 (m, 2H), 6.91 (d, 2H).

### Mode for Invention

### Preparation of dimethylaminoethyl 2[(2,6-dichlorophenyl)amino]benzene acetate.AcOH

2[(2,6-Dichlorophenyl)amino]benzene acetyl chloride hydrochloride (35.1 g, 0.1 mol) was dissolved in 100 ml of acetone. The mixture was cooled to 0°C. Dimethylaminoethanol (8.9 g, 0.1 mol) were added into the reaction mixture. Sodium bicarbonate (20 g) and water (100 ml) are added into the mixture. The mixture is stirred for 3 hours at RT. The solution is evaporated to dryness. Acetone (100 ml) is added into the residue. The solid side product was removed by filtration and washed with acetone (3 x 30 ml). 6 g of acetic acid is added into the reaction mixture with stirring. The organic solution was evaporated off. After drying, it yielded 38 g of the desired product (88.9 %). Hygroscopic product; Solubility in water: 410 mg/ml; Elementary analysis: C₂₀H₂₄Cl₂N₂O₄; MW: 427.32. Calculated % C: 56.21; H: 5.66; Cl: 16.59, N: 6.56; O: 14.98; Found % C: 56.18; H: 5.68; Cl: 16.56, N: 6.55; O: 15.03. ¹H-NMR (400 MHz, CDCl₃): δ: 2.21 (s, 3H), 2.91 (s, 6H), 3.50 (s, 2 H), 3.52 (m, 2H), 3.81 (b, 1H), 4.51 (t, 2H), 6.32 (d, 1H), 6.50 (m, 2 H), 6.78 (b, 1H), 6.82 (m, 2H), 6.91 (d, 2H).

### Preparation of S-dimethylaminoethyl 2[(2,6-dichlorophenyl)amino]benzene acetate.AcOH

2[(2,6-Dichlorophenyl)amino]benzene acetyl chloride hydrochloride (35.1 g, 0.1 mol) was dissolved in 100 ml of acetone. The mixture was cooled to 0°C. Dimethylaminoethyl mercaptan (9.3 g, 0.1 mol) were added into the reaction mixture. Sodium bicarbonate (20 g) and water (100 ml) are added into the mixture. The mixture is stirred for 3 hours at RT. The solution is evaporated to dryness. Acetone (100 ml) is added into the residue. The solid side product was removed by filtration and washed with acetone (3 x 30 ml). 6 g of acetic acid is added into the reaction mixture with stirring. The organic solution was evaporated off. After drying, it yielded 40 g of the desired product (90.2 %). Hygroscopic product; Solubility in water: 410 mg/ml; Elementary analysis: C₂₀H₂₄Cl₂N₂O₃S; MW: 443.39. Calculated % C: 54.18; H: 5.46; Cl: 15.99, N: 6.32; O: 10.83, S: 7.22; Found % C: 54.16; H: 5.48; Cl: 15.97, N: 6.31; O: 10.86, S: 7.23. ¹H-NMR (400 MHz, CDCl₃): δ: 2.21 (s, 3H), 2.91 (s, 6H), 3.31 (t, 2H), 3.66 (s, 2 H), 3.91 (m, 2H), 3.93 (b, 1H), 6.32 (d, 1H), 6.50 (m, 2 H), 6.78 (b, 1H), 6.82 (m, 2H), 6.91 (d, 2H).

### Preparation of N-dimethylaminoethyl 2[(2,6-dichlorophenyl)amino]benzene acetamide.AcOH

2[(2,6-Dichlorophenyl)amino]benzene acetyl chloride hydrochloride (35.1 g, 0.1 mol) was dissolved in 100 ml of acetone. The mixture was cooled to 0°C. Dimethylaminoethylamine (8.9 g) added into the reaction mixture. Sodium bicarbonate (20 g) and water (100 ml) are added into the mixture. The mixture is stirred for 3 hours at RT. The solution is evaporated to dryness. Acetone (100 ml) is added into the residue. The solid side product was removed by filtration and washed with acetone (3 x 30 ml). 6 g of acetic acid is added into the reaction mixture with stirring. The organic solution was evaporated off. After drying, it yielded 40 g of the desired product (93.8 %). Hygroscopic product; Solubility in water: 450 mg/ml; Elementary analysis: C₂₀H₂₅Cl₂N₂O MW: 426.34. Calculated % C: 56.34; H: 5.91; Cl: 16.63, N: 9.86; O: 11.26; Found % C: 56.31; H: 5.5.94; Cl: 16.61, N: 9.84; O: 11.30.¹H-NMR (400 MHz, CDCl₃): δ: 2.21 (s, 3H), 2.91 (s, 6H), 3.44 (s, 2 H), 3.51 (t, 2H), 3.64 (t, 2H), 3.93 (b, 1H), 6.32 (d, 1H), 6.50 (m, 2 H), 6.78 (b, 1H), 6.82 (m, 2H), 6.91 (d, 2H), 8.0 (b, 1H).

### Preparation of dipropylaminoethyl 2[(2,6-dichlorophenyl)amino]benzene acetate.AcOH

31.8 g (0.1 mol) of sodium 2[(2,6-dichlorophenyl)amino] benzene acetate was suspended in 180 ml of chloroform. 28.8 g (0.1 mol) of dipropylaminoethyl bromide.HBr was added into the mixture and the mixture was stirred for 5 hours at RT. 8.2 g (0.1 mol) of sodium acetate was added into the reaction mixture with stirring. The mixture is stirred for 2 hours. The solid was removed by filtration and washed with chloroform (3 x 50 ml). The solution is concentrated in vacuo to 100 ml. Then 300 ml of hexane was added into the solution. The solid product was collected by filtration and washed with hexane (3 x 100 ml). After drying, it yielded 41 g of the desired product (87%). Hygroscopic product; Solubility in water: 300 mg/ml; Elementary analysis: C₂₄H₃₂Cl₂N₂O₄; MW:483.43 Calculated % C: 59.63; H: 6.67; Cl: 14.67; N: 5.79; O: 13.24; Found % C: 59.60; H: 6.70; Cl: 14.65, N: 5.78; O: 13.27. ¹H-NMR (400 MHz, CDCl₃): δ: 0.97 (t, 6H), 1.78 (m, 4H), 2.21 (s, 3H), 3.24 (t, 4H), 3.50 (s, 2 H), 3.52 (m, 2H), 3.81 (b, 1H), 4.51 (t, 2H), 6.34 (d, 1H), 6.50 (m, 2 H), 6.78 (b, 1H), 6.82 (m, 2H), 6.91 (d, 2H).

### Preparation of dipropylaminoethyl dipropylaminoethyl 2[(2,6-dichlorophenyl)amino]benzene acetate.AcOH

60 g of Polymer-bound triethylamine (3 mmol/g, 100-200 mesh) was suspended in 180 ml of chloroform. 31.8 g (0.1 mol) of 2[(2,6-dichlorophenyl)amino] benzene acetic acid was added into the mixture with stirring. 43 g (0.15mol) of dipropylaminoethyl bromide.HBr was added into the mixture and the mixture was stirred for 5 hours at RT. The polymer is removed by filtration and washed with tetrahydrofuran (3 x 50 ml). 8.2 g (0.1 mol) of sodium acetate was added into the reaction mixture with stirring. The mixture is stirred for 2 h. The solid was removed by filtration and washed with chloroform (3 x 50 ml). The solution is concentrated in vacuo to 100 ml. Then 300 ml of hexane was added into the solution. The solid product was collected by filtration and washed with hexane (3 x 100 ml). After drying, it yielded 45 g of the desired product (93.2%). Hygroscopic product; Solubility in water: 300 mg/ml; Elem entary analysis: C₂₄H₃₂Cl₂N₂O₄; MW: 483.43 Calculated % C: 59.63; H: 6.67; Cl: 14.67; N: 5.79; O: 13.24; Found % C: 59.60; H: 6.70; Cl: 14.65, N: 5.78; O: 13.27. ¹H-NMR (400 MHz, CDCl₃): δ: 0.97 (t, 6H), 1.78 (m, 4H), 2.21 (s, 3H), 3.24 (t, 4H), 3.50 (s, 2 H), 3.52 (m, 2H), 3.81 (b, 1H), 4.51 (t, 2H), 6.34 (d, 1H), 6.50 (m, 2 H), 6.78 (b, 1H), 6.82 (m, 2H), 6.91 (d, 2H).

### Industrial Applicability

The pro-drugs of the general formula (1) 'Structure 1' are superior to diclofenac. They may be used medicinally in treating any diclofenac-treatable conditions in humans or animals. They can be used for the relief of signs and symptoms of rheumatoid arthritis, osteoarthritis and ankylosing spondylitis, for the treatment of dysmenorrhea. They can be used alone or as an adjunct in the treatment of biliary colic, fever, and episiotomy pain. They are also used in treatment of gout, acute migraine headaches, and renal colic and in the treatment of postoperative inflammation in patients who have undergone cataract extraction. They may be used in the prevention of cancer. Due to their very high membrane penetration rate, these pro-drugs can be used in treating asthma by inhalation to a host. They can be used to treat acne due to their anti-inflammatory properties. These pro-drugs are water-soluble neutral salt and can be tolerated very well by the eye. They can be used for treating eye inflammatory diseases, for treating of ocular pain after corneal surgery, for treating glaucoma or for treating ear inflammatory and/or painful conditions (otitis).

Further preferred embodiments are given in the following paragraphs:
[1] The compounds of the general formula (1) 'Structure 1', 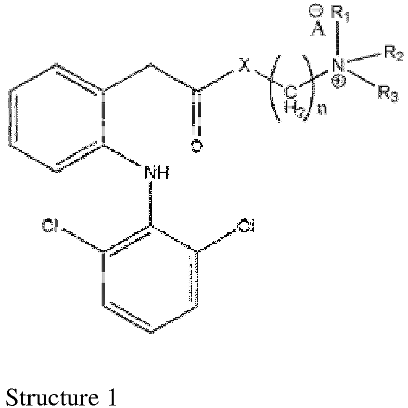 In which, R represents H, one of any alkyl, alkyloxy, alkenyl, or alkynyl residues having 1 to 12 carbon atoms, or aryl residues; R represents H, one of any alkyl, alkyloxy, alkenyl or alkynyl residues having 1 to 12 carbon atoms, or aryl residues; R represents H, one of any alkyl, alkyloxy, alkenyl, or alkynyl residues having 1 to 12 carbon atoms, or aryl residues; X represents O, S or NH; A represents Cl", Br", F , I, AcO , acetylsalicylate, citrate, or any negative ions; and n=0,1,2,3,4,5,6,7,8,9,10 All R groups may include C, H, O, S, N atoms and may have single, double, and treble bonds. Any CH groups may be replaced with O, S, or NH.
[2] Process for the preparation of compounds of the general formula (1) 'Structure 1' according to Embodiment 1.
[3] Compounds of the general 'Structure 1' or a composition comprising of at least one compound of the general formula (1) 'Structure 1', as an active ingredient, according to Embodiment 1, where they can be administered orally or transdermally, for treating any diclofenac-treatable conditions in humans or animals. The diclofenac-treatable conditions include, but are not limited to, pain from a toothache, headache, and arthritis and other inflammatory pain, fever, cancer, dysmenorrhea, acute migraine headache.
[4] Methods for treating any diclofenac-treatable conditions in humans or animals by administering transdermally to any part of body (in the from of a solution, spray, lotion, ointment, emulsion or gel) to deliver therapeutically effective plasma levels of compounds of the general formula (1) 'Structure 1' or a composition comprising of at least one compound of the general formula (1)'Structure 1', as an active ingredient, according to Embodiment 1.
[5] Methods for topically treating pain such as a headache, toothache, and muscle pain, and arthritis and other inflammatory pain in humans or animals by administering to the inflamed area a therapeutically effective amount of the compounds of the general formula (1) 'Structure 1' or a composition comprising of at least one compound of the general formula (1) 'Structure 1', as an active ingredient, according to Embodiment 1.
[6] Compounds of the general formula (1) 'Structure 1' or a composition comprising of at least one compound of the general formula (1) 'Structure 1', as an active ingredient, according to Embodiment 1, may be administered transdermally, for treating acne, sunburn or other skin disorders in the form of a solution, spray, lotion, ointment, emulsion or gel.
[7] Compounds of the general 'Structure 1' or a composition comprising of at least one compound of the general formula (1) 'Structure 1', as an active ingredient, according to Embodiment 1, are administered by spraying to through the mouth or nose or other parts of body for treating asthma.
[8] Compounds of the general 'Structure 1' or a composition comprising of at least one compound of the general formula (1) 'Structure 1', as an active ingredient, according to Embodiment 1, for treating any eye inflammatory diseases in humans or animals.
[9] Transdermal therapeutic application systems of Compounds of the general formula (1) 'Structure 1' or a composition comprising of at least one compound of the general formula(1) 'Structure 1', as an active ingredient, according to embodiment 1 for treating any diclofenac-treatable conditions in humans or animals. These systems can be a bandage or a patch comprising of one active substance- containing matrix layer and an impermeable backing layer. The most preferable system is an active substance reservoir, which has a permeable bottom facing the skin. By controlling the rate of release, this system enables the diclofenac to reach constantly optimal therapeutic blood levels to increase effectiveness and reduce the side effects of diclofenac.

## Claims

1. A compound of the general Structure 1 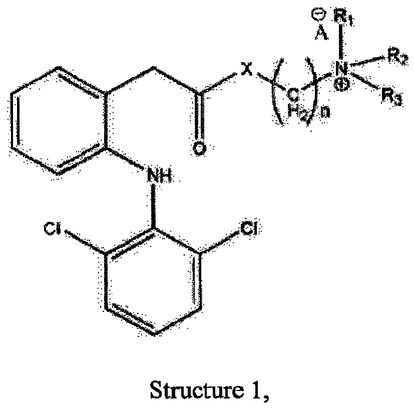 wherein
all R groups may include C, H, O, S, N atoms and may have single, double, and triple bonds; and any CH₂ groups may be replaced with O, S, or NH;
X represents O or S,
A⁻ represents any negative ion, and
n=0,1,2,3,4,5,6,7,8,9,10.

2. The compound according to claim 1, wherein A- is selected from the group consisting of Cl⁻, Br⁻, F⁻, I⁻, and AcO⁻.

3. A composition comprising one or more compounds according to claim 1.

4. The composition according to claim 3, wherein the composition comprises water.

5. The compound according to claim 1 or 2 or the composition according to claim 3 or 4, for use in a method of treating asthma or a condition treatable by diclofenac in human or animal.

6. The compound or composition for use according to claim 5, wherein the treated condition is selected from the group consisting of pain, inflammatory diseases, cancers, fever, skin disorders, toothache, headache, muscle pain, arthritis, inflammatory pain, dysmenorrhea, acute migraine headache, acne, sunburn, and eye inflammatory diseases.

7. The compound or composition for use according to claim 5 or 6, wherein the compound or composition is administered to deliver a therapeutically effective plasma level of the compounds or the composition.

8. The compound or composition for use according to any of claims 5 to 7, wherein the compound or composition is administered in the form of a solution, spray, lotion, ointment, emulsion, or gel.

9. The compound or composition for use according to any of claims 5 to 8, wherein the compound or composition is administered orally, transdermally, or topically.

10. The compound or composition for use according to any of claims 5 to 8, wherein the compound or composition is administered to a body part of the human or animal selected from the group consisting of skin, inflamed area, mouth, and nose.

11. Transdermal therapeutic application systems comprising a compound according to claim 1 or 2, or a composition according to claim 3 or 4.

12. The transdermal therapeutic application systems according to claim 11, **characterized in that** the system is a bandage or a patch comprising an active-substance-containing matrix layer, and an impermeable backing layer.

13. The transdermal therapeutic application systems according to claim 11 or 12, **characterized by** having an active substance reservoir, which has a permeable bottom facing the skin.

14. The transdermal therapeutic application systems according to any of claims 11 to 13, **characterized by** a controlling means for controlling the rate of release, enabling the system to reach constantly optimal therapeutic blood levels.

15. The transdermal therapeutic application systems according to any of claims 11 to 14, for use in a method of treating asthma or a condition treatable by diclofenac in human or animal.
